# EUROPEAN PATENT SPECIFICATION

(11) **EP 2 478 859 B1**
(45) Date of publication and mention of the grant of the patent: **29.03.2017**
(21) Application number: 12152164.5
(22) Date of filing: 23.01.2012
(51) Int. Cl.: A61B 17/34, A61B 17/00

(54) **Inflatable access assembly**
Aufblasbare Zugangsanordnung
Ensemble d'accès gonflable

(30) Priority: 24.01.2011 US 201161435426 P; 05.01.2012 US 201213343826
(43) Date of publication of application: 25.07.2012
(73) Proprietor: Covidien LP, Mansfield, MA 02048 (US)
(72) Inventor: Kleyman, Gennady, Brooklyn, NY New York 11230 (US)
(74) Representative: Soames, Candida Jane

(56) References cited:
- EP-A1- 2 044 889
- EP-A1- 2 289 438
- WO-A1-00/54675
- US-A1- 2009 221 966
- US-B1- 7 850 600

## Description

### BACKGROUND

### Technical field

The present disclosure relates to compressible access assemblies for use in surgical procedures. More particularly, the present disclosure relates to compressible access assemblies including an inflatable sleeve.

### Background of Related Art

Access assemblies configured for reception through an opening or incision into a body cavity are known, as are methods of inserting the access assemblies therethrough. Traditional access assemblies include a rigid cannula that is received through the tissue of the body wall into the body cavity. Endoscopic, laparoscopic and other suitable instruments may then be directed through a housing located on the proximal end of the cannula to access the body cavity in a sealing manner.

Compressible assemblies configured for accessing a body cavity and permitting reception of instruments therethrough in sealing manner are also known. Such compressible assemblies are composed of silicone, thermoplastic elastomers (TPE), rubber, foam, gel and other compressible materials and are configured to be compressed to facilitate insertion into an incision. Typically, such assemblies are deformed by a surgeon using his/her fingers or with the assistance of a grasping device, i.e., forceps. Compression of the assembly reduces the profile of the assembly, thereby facilitating reception of the assembly into the incision. Upon release of the compressive force, the compressed assembly returns to an uncompressed configuration.

Applying a compressive force to the compressive access assemblies, whether by hand or using an insertion device, excessive handling may damage the assembly. Additionally, maintaining the compressive force on the access assembly during installation and reapplying the compressive force during removal of the access assembly may result in damage to surrounding tissue.

Therefore, it is desirable to provide a compressible access assembly which is capable of being received through an opening and removed therefrom with limited compressive force.

US7850600 discloses a surgical access assembly comprising an inflatable sleeve and a disc which may be inserted into an incision in a patient's body. Instruments may be passed through ports in the disc. The device is made foldable to allow the assembly to be inserted into the incision whereupon the sleeve and disc are inflated. The preamble of claim 1 is based on this document.

WO 00/54675 discloses an access assembly comprising a sleeve with an external and internal bladder. The assembly is used with a distal ring and proximal ring to retain the access assembly in position with the bladders providing sealing.

EP 2044889 discloses a seal anchor for use in surgical procedures comprising a seal anchor member made of compressible material having lumens through which instruments may be inserted. The member is compressed to permit insertion into an incision in tissue. US2009/0221966 discloses a a surgical access device provided with an inflatable sleeve which depends from a access port assembly through which instruments may be inserted.

### SUMMARY

According to, the present invention there is provided an access assembly as recited in claim 1. Preferred embodiments are disclosed in the dependent claims.

The inflatable sleeve may include upper and lower rims. One or both of the inflatable sleeve and base may be composed of at least one of silicone, thermoplastic elastomers (TPE), rubber, foam, gel. The base may be selectively secured about the base, or the base may be securely affixed to the base by adhesive, welding, bonding or mechanical fasteners. The base may include at least one valve member in the at least first lumen.

In an embodiment, the opening in the tissue is an incision. Alternatively, the opening in tissue may be a natural orifice. The inflatable sleeve may include an inflated condition and a deflated condition. Also, the inflatable sleeve includes at least a first inflatable portion and a second inflatable portion. In addition, the inflatable sleeve may include a third inflatable portion. The inflatable sleeve may include an inflation port configured for operable connection with a source of inflation fluid.

### DESCRIPTION OF THE DRAWINGS

Embodiments of a flexible access assembly according to the claimed invention and examples, useful for the understanding of the invention, are disclosed herein with reference to the drawings, wherein:
FIG. 1 is a perspective view of an example of an access assembly according to the present disclosure operably connected to a source of inflation fluid;
FIG. 2 is a cross-sectional side view of the access assembly of FIG. 1, in a deflated condition;
FIG. 3 is a cross-sectional side view of the access assembly of FIG. 1, in an inflated condition;
FIG. 4 is a cross-sectional side view of the access assembly of FIG. 1, in a deflated condition prior to insertion through an incision in tissue;
FIG. 5 is a cross-sectional side view of the access assembly of FIG. 1, in a deflated and compressed condition;
FIGS. 6 is a cross-sectional side view of the access assembly of FIG. 5, in a deflated condition upon insertion through an incision in tissue;
FIG. 7 is a cross-sectional side view of the access assembly of FIG. 6, in an inflated condition and securely received within the incision in tissue;
FIG. 8 is a cross-sectional side view of the access assembly of FIG. 7, including surgical devices received therethrough;
FIG. 9 is a cross-sectional side view of an embodiment of an access assembly according to the present invention; and
FIG. 10 is a cross-sectional side view of another example of an access assembly

### DETAILED DESCRIPTION

Embodiments of the presently disclosed inflatable access assemblies and further examples, useful for the understanding of the invention, will now be described in detail with reference to the drawings wherein like numerals designate identical or corresponding elements in each of the several views. As is common in the art, the term "proximal" refers to that part or component closer to the user or operator, i.e. surgeon or physician, while the term "distal" refers to that part or component further away from the user. Although the access assemblies of the present disclosure will be described as relates to accessing an abdominal cavity through an incision in the abdominal wall, the access assemblies of the present disclosure may be modified for use in other closed procedures, i.e., laparoscopic, arthroscopic, endoscopic. Furthermore, the access assemblies of the present disclosure may be modified for use in accessing internal cavities or lumen through natural orifices, e.g., anus, vagina.

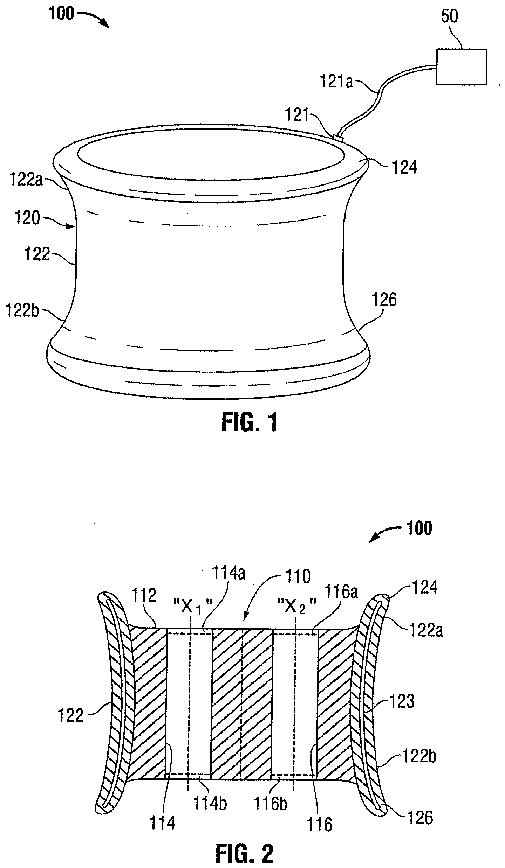

Referring initially to FIG. 1, an inflatable access assembly according to an example is shown generally as access assembly 100. Access assembly 100 is configured for insertion through an opening in tissue, i.e., an incision, such that after insertion, access assembly 100 creates a seal within the opening through which a surgeon may insert and manipulate one or more surgical instruments to complete a procedure.

With reference to FIGS. 1-3, access assembly 100 includes a base 110 and an inflatable sleeve or flange 120. Base 110 and sleeve 120 may be formed of various materials, such as, for example, silicone, thermoplastic elastomers (TPE), rubber, foam, gel, etc. Base 110 and sleeve 120 may be constructed from the same or different materials. In one example, base 110 includes a TPE material that is infused with an inert gas, e.g. CO₂ or Nitrogen, to form a foam structure, while sleeve 120 is formed of an elastic material that permits expansion of sleeve 120 upon inflation. Base 110 and sleeve 120 may be integrally formed. Alternatively, sleeve 120 is secured to base 110 with adhesive, welding, bonding, fasteners or any other suitable method. Sleeve 120 may be coated with a lubricant, e.g. Parylene N or C, in order to create a lubricious surface. Various other coatings, e.g., hydrophilic, hydrophobic, bio-agents, anti-infection, analgesic, may also be employed to improve the characteristics of access assembly 100 or to adapt access assembly 100 for a specific procedure.

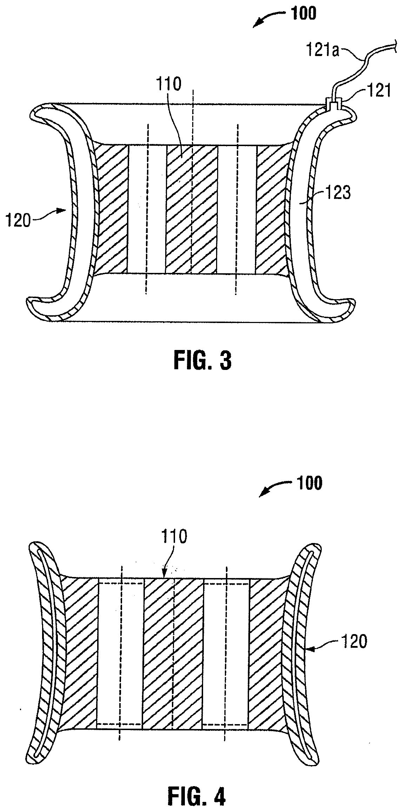

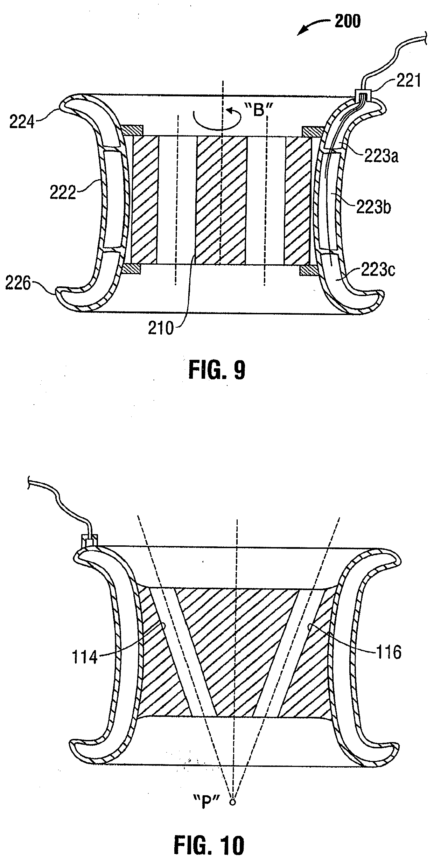

With particular reference now to FIGS. 2 and 3, base 110 of access assembly 100 includes a substantially cylindrical body 112 defining one or more lumens 114, 116 extending therethrough. As shown, base 110 includes two lumens 114, 116 having substantially similar size and shape for receiving instruments of substantially similar diameter. Alternatively, lumens 114, 116 may have different sizes and/or shapes for receiving instruments of different configurations. In one example, base 110 defines a single lumen for receiving a single, large instrument. Lumens 114, 116 extend through base 110 and define longitudinal axes, "x1", "x2", respectively, configured to receive surgical instruments, cannula assemblies, valve assemblies and/or insufflation apparatus in a sealed manner. In one example (FIG. 10), first and second lumens 114, 116 are each provided at an angle such that instruments directed therethrough intersect at a point "P" a predetermined distance from a distal end thereof. Either or both of lumens 114, 116 may include one or more valve members 114a, 114b, 116a, 116b (shown in phantom, FIG. 2), respectively, to permit sealed reception of surgical devices "D1", "D2" therethrough. Lumens 114, 116 may include a protective lining (not shown) extending along at least a length thereof to prevent tearing of base 110 as surgical devices "D1", "D2" are received and manipulated therethrough. Lumens 114, 116 may also be coated with a lubricant to assist in insertion of surgical devices "D1", "D2" therethrough.

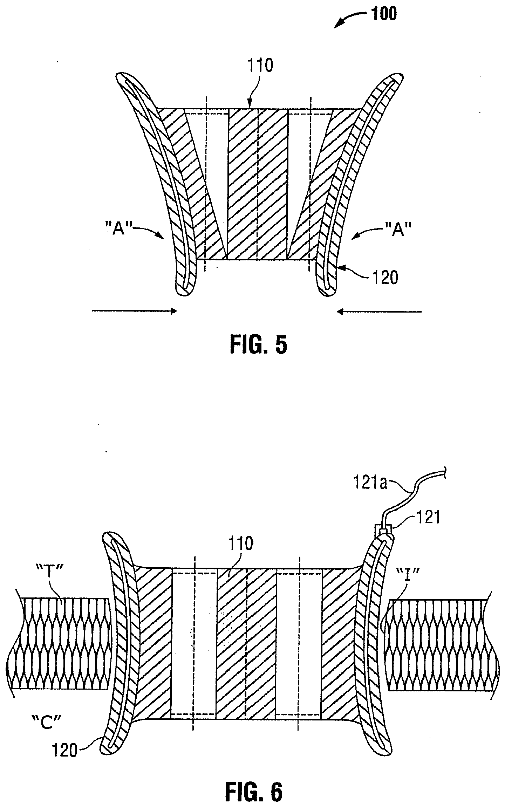

Still referring to FIGS. 2 and 3, access assembly 100 further includes inflatable sleeve 120 configured to be securely received about base 110. As discussed above, sleeve 120 may be integrally formed with base 110, or instead, may be securely attached thereto with adhesive, welding, bonding, fasteners or any other suitable method. Sleeve 120 includes a central portion 122 having an upper rim 124 located at a proximal end 122a thereof and a lower rim 126 located at a distal end 122b thereof. Central portion 122 defines a cavity 123 for receiving an inflation fluid. In use, central portion 122 is configured to span the thickness of tissue "T" (FIG. 6), however, in a deflated condition, central portion 122 may be as long as base 110. As will be discussed in further detail below, in an inflated condition, upper rim 124 and lower rim 126 of sleeve 120 aid in preventing movement of access assembly 100 longitudinally through incision "I" (FIG. 6). As the thickness of tissue depends on the body composition of the patient and the location through which the underlying cavity is being accessed, the length and size of access assembly 100, generally, and inflatable sleeve 120, specifically, may be modified to suit a given procedure. In this manner, an adult patient having fatty abdominal tissue may require an access assembly having a longer central portion 122 then an access assembly sized for a child.

With reference briefly back to FIG. 1, inflatable sleeve 120 includes an inflation port 121 on a proximal end thereof operably connected to a source of inflation fluid 50 via tubing 121a. Tubing 121a may be selectively or permanently secured to port 121. Source of inflation fluid 50 may include a hand pump, a portable gas source, i.e., gas cartridge and dispenser, an air compressor or any other method for providing fluid to inflatable sleeve 120. Although the inflation fluid used to fill inflatable sleeve 120 is typically gaseous, it is envisioned that sleeve 120 may be inflated with any suitable fluid, including, CO₂, Nitrogen, saline or a combination thereof. Inflation port 121 may include a valve (not shown) to permit release of the inflation gas upon completion of a procedure.

Referring now to FIG. 2, in a deflated condition, inflatable sleeve 120 of access assembly 100 is configured to be inserted through an incision "I" (FIG. 6) in tissue "T" (FIG. 6). In the deflated condition, upper and lower rims 124, 126 formed on proximal and distal ends 122a, 122b of central portion 122 become less pronounced, thereby facilitating insertion of access assembly 100 through incision "T". As will be discussed with reference to FIG. 5, base 110 may further be compressed to facilitate insertion of access assembly 100 through incision "I".

Referring now to FIG. 3, in an inflated condition, inflatable sleeve 120 of access assembly 100 is configured to be secured within incision "I" (FIG. 6) formed in tissue "T" (FIG. 6). Inflation of inflatable sleeve 120 causes central portion 122 to expand and/or stretch. In this manner, upper and lower rims 124, 126 become pronounced. Inflatable sleeve 120 may be inflated as much or as little as necessary to ensure a seal with tissue "T".

The use of access assembly 100 will now be described with reference to FIGS. 4-8. The following discussion will include using access assembly 100 for accessing a body cavity "C" through an incision "I". As discussed above, access assembly 100 may be used for accessing other cavities or lumen through other openings in the body, including naturally occurring orifices, e.g., anus.

Referring initially to FIG. 4, if not provided in a deflated state, inflatable sleeve 120 of access assembly 100 is deflated. An incision "I" (FIG. 6) is then created through tissue "T" (FIG. 6) to access body cavity "C". Although capable of reception through incision "I" when inflatable sleeve 120 is deflated, access assembly 100 may be compressed to further facilitate insertion of access assembly 100 through incision "I".

With reference now to FIG. 5, the compressible nature of base 110 permits compression of access assembly 100 to further facilitate insertion thereof into incision "I". As shown, a distal end of access assembly 100 is pressed inwardly, as indicated by arrows "A", to compress base 110 and further narrow access assembly 100 for insertion through incision "I". Although inflatable sleeve 120 is shown as being deflated during insertion of access assembly 100 into incision "I", it is envisioned that inflatable sleeve 120 may remain inflated or partially inflated during insertion. In this manner, body 110 may be compressed sufficiently such that inflatable sleeve 120 need not be deflated to permit reception of access assembly 100 through incision "I".

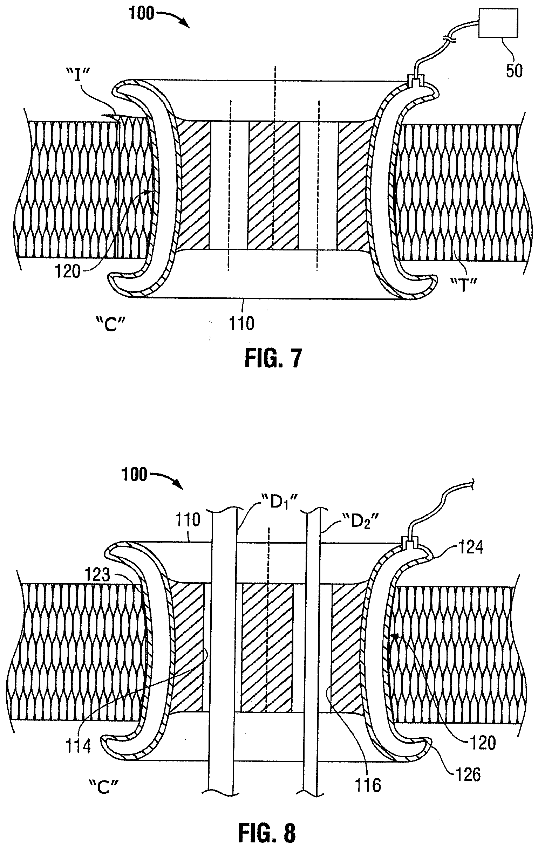

Turning to FIG. 7, once received through incision "I", base 110 is permitted to return to an initial, uncompressed condition. Inflatable sleeve 120 is then inflated to secure access assembly 100 within incision "I" and thereby seal body cavity "C". Inflatable sleeve 120 may be inflated as much or as little as necessary to secure and seal access assembly 100 within incision "I".

With reference now to FIG. 8, access assembly 100 may be operated in a traditional manner. As discussed above, each of lumen 114, 116 are configured to receive one or more surgical devices "D1", D2" therethrough. Removal of access assembly 100 from within incision "I" occurs in the reverse order of insertion. Inflatable sleeve 120 is first deflated. Then base 110 may be compressed before access assembly 100 is withdrawn from incision "I". Alternatively, inflatable sleeve 120 may remain inflated as base 110 is compressed to permit the withdrawal of access assembly 100 from incision "I". Once access assembly 100 is removed from incision "I", incision "I" is closed in a conventional manner.

Turning now to FIG. 9, an access assembly according to the embodiment of the present invention is shown generally as access assembly 200. Access assembly 200 is substantially similar to access assembly 100, and therefore, will only be described as relates to the difference therebetween. Access assembly 200 includes a base 210 and a sleeve 220. Base 210 is configured for rotational movement relative to inflatable sleeve 220, as indicated by arrow "B". In one embodiment, base 210 is configured to be selectively received within a central portion 222 of inflatable sleeve 220.

With reference still to FIG. 9, inflatable sleeve 220 defines a first inflation cavity 223a located in an upper rim 224, a second inflation cavity 223b located in a central portion 222, and a third inflation cavity 223c located in lower rim 226. Each of first, second and third cavities 223a, 223b, 223c are individually connected to a source of inflation fluid 50 (FIG. 1) through inflation port 221. Thus, upper rim 224, lower rim 226 and central portion 222 of sleeve 220 may be selectively inflated depending on the configuration and location cavity being accessed. In this manner, access assembly 200 may be more securely received with incision "I".

It will be understood that various modifications may be made to the embodiments disclosed herein. Therefore, the above description should not be construed as limiting, but merely as exemplifications of particular embodiments. Those skilled in the art will envision other modifications within the scope of the claims appended hereto. For example, either of the base or inflatable sleeve may have a groove or lip, and the other of the base or inflatable sleeve may have a corresponding lip or groove, and the groove of one is configured to engage the lip of the other to join the base of the inflatable sleeve.

## Claims

1. An access assembly (200) comprising:
a base (210) including a substantially cylindrical compressible member defining at least a first lumen (114, 116) extending therethrough; and
an elongated, generally cylindrical inflatable sleeve (222) connected to and positioned circumferentially around the base, the inflatable sleeve having an inner surface to which the base is generally transverse, the inflatable sleeve being configured to selectively retain the access assembly within an opening in tissue **characterised in that** the inflatable sleeve includes at least a first inflatable portion (223a) and a second inflatable portion (223b) wherein the inflatable portions are selectively inflatable.

2. The access assembly of Claim 1, wherein the inflatable sleeve includes upper (224) and lower rims (226).

3. The access assembly of Claim 1 or Claim 2, wherein at least on of the inflatable sleeve and base is composed of at least one of silicone, thermoplastic elastomers (TPE), rubber, foam, gel.

4. The access assembly of any preceding Claim, wherein the sleeve is selectively secured about the base.

5. The access assembly of Claim 4, wherein the sleeve is securely affixed to the base by adhesive, welding, bonding or mechanical fasteners.

6. The access assembly of any preceding Claim, wherein the base includes at least one valve member (114a, 114b, 116a, 116b) in the at least first lumen.

7. The access assembly of any preceding Claim, wherein the opening in the tissue is an incision.

8. The access assembly of any of Claims 1 to 6, wherein the opening in tissue is a natural orifice.

9. The access assembly of any preceding Claim, wherein the inflatable sleeve includes an inflated condition and a deflated condition.

10. The access assembly of any preceding claim, wherein the inflatable sleeve further includes a third inflatable portion (223c).

11. The access assembly of any preceding Claim, wherein the inflatable sleeve includes an inflation port (221) configured for operable connection with a source of inflation fluid.

## Patentansprüche

1. Zugangsanordnung (200), umfassend:
eine Basis (210) einschließlich eines im Wesentlichen zylindrischen, komprimierbaren Elements, das wenigstens ein erstes Lumen (114, 116) definiert, das sich dort hindurch erstreckt; und
eine längliche, im Allgemeinen zylindrische, aufblasbare Hülle (222), die mit der Basis verbunden und umlaufend darum positioniert ist, wobei die aufblasbare Hülle eine Innenfläche hat, zu der die Basis im Allgemeinen transversal ist, wobei die aufblasbare Hülle dazu konfiguriert ist, die Zugangsanordnung selektiv innerhalb einer Öffnung in Gewebe zu halten, **dadurch gekennzeichnet, dass** die aufblasbare Hülle wenigstens einen ersten aufblasbaren Abschnitt (223a) und einen zweiten aufblasbaren Abschnitt (223b) umfasst, wobei die aufblasbaren Abschnitte selektiv aufblasbar sind.

2. Zugangsanordnung nach Anspruch 1, wobei die aufblasbare Hülle obere (224) und untere Ränder (226) umfasst.

3. Zugangsanordnung nach Anspruch 1 oder Anspruch 2, wobei wenigstens eines von der aufblasbaren Hülle und der Basis aus wenigstens einem von Silikon, thermoplastischen Elastomeren (TPE), Gummi, Schaum, Gel besteht.

4. Zugangsanordnung nach einem vorangegangenen Anspruch, wobei die Hülle selektiv um die Basis herum gesichert ist.

5. Zugangsanordnung nach Anspruch 4, wobei die Hülle durch Klebstoff, Schweißen, Verbindung oder mechanische Befestigungsmittel sicher an der Basis befestigt ist.

6. Zugangsanordnung nach einem vorangegangenen Anspruch, wobei die Basis wenigstens ein Ventilelement (114a, 114b, 116a, 116b) in dem wenigstens ersten Lumen umfasst.

7. Zugangsanordnung nach einem vorangegangenen Anspruch, wobei die Öffnung in dem Gewebe ein Einschnitt ist.

8. Zugangsanordnung nach einem der Ansprüche 1 bis 6, wobei die Öffnung in Gewebe eine natürliche Öffnung ist.

9. Zugangsanordnung nach einem vorangegangenen Anspruch, wobei die aufblasbare Hülle einen aufgeblasenen Zustand und einen entleerten Zustand umfasst.

10. Zugangsanordnung nach einem vorangegangenen Anspruch, wobei die aufblasbare Hülle weiter einen dritten aufblasbaren Abschnitt (223c) umfasst.

11. Zugangsanordnung nach einem vorangegangenen Anspruch, wobei die aufblasbare Hülle eine Aufblasöffnung (221) umfasst, die für eine betriebsbereite Verbindung mit einer Quelle von einem Aufblasfluid konfiguriert ist.

## Revendications

1. Ensemble d'accès (200) comprenant :
une base (210) incluant un élément compressible sensiblement cylindrique définissant au moins une première lumière (114, 116) s'étendant à travers cette dernière ; et
un manchon gonflable allongé globalement cylindrique (222) relié à et positionné de manière circonférentielle autour de la base, le manchon gonflable ayant une surface intérieure à laquelle la base est globalement transversale, le manchon gonflable étant configuré pour retenir de manière sélective l'ensemble d'accès à l'intérieur d'une ouverture dans le tissu **caractérisé en ce que** le manchon gonflable inclut au moins une première portion gonflable (223a) et une deuxième portion gonflable (223b) dans lesquelles les portions gonflables sont gonflables de manière sélective.

2. Ensemble d'accès selon la revendication 1, dans lequel le manchon gonflable inclut des rebords supérieur (224) et inférieur (226).

3. Ensemble d'accès selon la revendication 1 ou la revendication 2, dans lequel au moins un du manchon gonflable et de la base est composé d'au moins un de silicone, d'élastomères thermoplastiques (TPE), de caoutchouc, de mousse, de gel.

4. Ensemble d'accès selon l'une quelconque des revendications précédentes, dans lequel le manchon est fixé de manière sélective autour de la base.

5. Ensemble d'accès selon la revendication 4, dans lequel le manchon est fixé de manière sûre à la base par adhésif, soudage, liaison ou attaches mécaniques.

6. Ensemble d'accès selon l'une quelconque des revendications précédentes, dans lequel la base inclut au moins un élément formant soupape (114a, 114b, 116a, 116b) dans l'au moins première lumière.

7. Ensemble d'accès selon l'une quelconque des revendications précédentes, dans lequel l'ouverture dans le tissu est une incision.

8. Ensemble d'accès selon l'une quelconque des revendications 1 à 6, dans lequel l'ouverture dans le tissu est un orifice naturel.

9. Ensemble d'accès selon l'une quelconque des revendications précédentes, dans lequel le manchon gonflable inclut un état gonflé et un état dégonflé.

10. Ensemble d'accès selon l'une quelconque des revendications précédentes, dans lequel le manchon gonflable inclut en outre une troisième portion gonflable (223c).

11. Ensemble d'accès selon l'une quelconque des revendications précédentes, dans lequel le manchon gonflable inclut un orifice de gonflage (221) configuré pour un raccordement fonctionnel avec une source de fluide de gonflage.
